(19) Europäisches Patentamt
European Patent Office
Office européen des brevets

(11) Veröffentlichungsnummer: **0 014 840**
**B1**

(12) # EUROPÄISCHE PATENTSCHRIFT

(45) Veröffentlichungstag der Patentschrift: **06.01.82**

(51) Int. Cl.³: **C 07 C 49/567,** C 07 C 25/18, C 07 C 45/46, C 07 C 17/00, C 09 K 3/34

(21) Anmeldenummer: **80100194.2**

(22) Anmeldetag: **16.01.80**

(54) **Fluorphenyl-trans-cyclohexane, Verfahren zu ihrer Herstellung, diese enthaltende flüssigkristalline Dielektrika und elektrooptisches Anzeigeelement.**

(30) Priorität: **24.02.79 DE 2907332**

(43) Veröffentlichungstag der Anmeldung: **03.09.80 Patentblatt 80/18**

(45) Bekanntmachung des Hinweises auf die Patenterteilung: **06.01.82 Patentblatt 82/1**

(84) Benannte Vertragsstaaten: **AT CH DE FR GB IT NL**

(56) Entgegenhaltungen:
**DE-A-2 636 684**
**1977 SID INTERNATIONAL SYMPOSIUM DIGEST OF TECHNICAL PAPERS, erste Auflage, April 1977, Seite 77, Publisher: Lewis Winner, New York U.S.A**
**ANGEWANDTE CHEMIE, INTERNATIONAL EDITION IN ENGLISH, Band 16, Februar 77 Seite 100 Weinheim, DE. R. EIDENSCHINK et al.: »Substituted Phenylcyclohexanes — A New Class of Liquid-Crystalline Compounds«**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Dessauer Strasse 57, D-6110 Dieburg (DE)**
Erfinder: **Pohl, Ludwig, Dr., Hoffmannstrasse 54, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Fluorphenyl-trans-cyclohexane, Verfahren zu ihrer Herstellung, diese enthaltende flüssigkristalline Dielektrika und elektrooptisches Anzeigeelement

Für Flüssigkristall-Anzeigeelemente werden die Eigenschaften nematischer oder nematisch-cholesterischer flüssigkristalliner Materialien ausgenutzt, ihre optischen Eigenschaften wie Lichtdurchlässigkeit, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem nematisch-cholesterischen Phasenübergang.

Für die technische Anwendung dieser Effekte in Flüssigkristall-Anzeigeelementen werden flüssigkristalline Materialien benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Materialien eine flüssigkristalline Mesophase im Temperaturbereich von mindestens 0°C bis +50°C, bevorzugt von −10°C bis +70°C, und eine Viskosität bei Raumtemperatur von nicht mehr als 60 cP gefordert. Schließlich dürfen sie im Bereich des sichtbaren Lichts keine Eigenabsorption aufweisen, d. h., sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p′-disubstituierten Benzoesäurephenylester, die in der DE-OS 2 356 085 beschriebenen p,p′-disubstituierten Biphenylderivate oder die in der DE-OS 2 636 684 beschriebenen Phenylcyclohexanderivate.

In den genannten Verbindungsklassen wie auch in anderen bekannten Reihen von Verbindungen mit flüssigkristalliner Mesophase gibt es keine Einzelverbindungen, die in dem geforderten Temperaturbereich von 0°C bis 60°C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt bei einer tieferen oder etwa der gleichen Temperatur wie der Schmelzpunkt der niedriger schmelzenden Komponente liegt, während der Klärpunkt zwischen den Klärpunkten der Komponenten liegt. Als Komponenten mit höheren Schmelz- und Klärpunkten sind bisher beispielsweise 4,4″-disubstituierte p-Benzoyloxybenzoesäurephenylester der Formel (III)

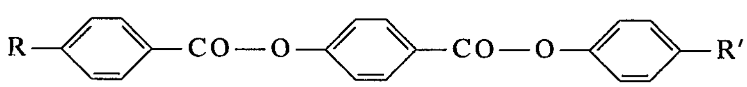
(III)

oder die Biphenylesterderivate der Formel (IV)

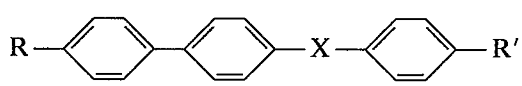
(IV)

worin

R und R′ Alkyl- oder Alkoxygruppen sind und
X eine Carbonyloxygruppe bedeutet,

verwendet worden. Die Verbindungen der Formel (III) haben jedoch keinen großen Anwendungsbereich gefunden, weil sie den sie enthaltenden flüssigkristallinen Dielektrika eine hohe Viskosität verleihen; dadurch werden die Schaltzeiten der damit hergestellten Flüssigkristall-Anzeigeelemente in unerwünschter Weise verlängert. Dieser Effekt ist zwar bei den Biphenylestern der Formel (IV) nicht so stark ausgeprägt, diese Verbindungen sind jedoch, vor allem bei tiefen Temperaturen, in den wichtigsten flüssigkristallinen Basismaterialien nicht so gut löslich wie es zu Anhebung des Klärpunkts erwünscht ist.

Der Erfindung liegt daher die Aufgabe zugrunde, flüssigkristalline Dielektrika mit einem breiten, die Raumtemperatur einschließenden Temperaturbereich der nematischen Phase und möglichst niedriger Viskosität zur Verfügung zu stellen.

Erfindungsgemäß wird diese Aufgabe dadurch gelöst, daß bekannten flüssigkristallinen Substanzen mit hoher Viskosität ein oder mehrere Fluorphenyl-trans-cyclohexane der Formel (I)

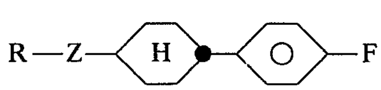
(I)

worin R eine Alkylgruppe mit 1 — 17 Kohlenstoffatomen und Z entweder eine Carbonylgruppe oder eine Methylengruppe bedeutet, als die Viskosität erniedrigende Komponenten zugemischt werden.

Die Fluorphenylcyclohexane der Formel (I) sind in der Regel monotrop flüssigkristallin, d. h. ihr Klärpunkt liegt bei tieferer Temperatur als ihr Schmelzpunkt. Ihre nematische Phase läßt sich daher nur in einer unterkühlten Schmelze beobachten, so daß sie ohne weitere Mischungskomponenten nicht als Dielektrika für Flüssigkristall-Anzeigeelemente verwendet werden können. Sie sind jedoch sehr gut in bekannten Flüssigkristallmischungen löslich und erniedrigen die Viskosität solcher Substanzen derart, daß sich mit den daraus erhaltenen Dielektrika Flüssigkristall-Anzeigeelemente mit bisher kaum erreichbar kurzen Schaltzeiten realisieren lassen.

Gegenstand der Erfindung sind somit die Fluorphenyl-trans-cyclohexane der Formel (I) sowie Verfahren zu ihrer Herstellung. Gegenstand der Erfindung ist weiterhin die Verwendung der Fluorphenylcyclohexane der Formel (I) in Gemisch mit anderen flüssigkristallinen Substanzen als Dielektrika in Flüssigkristall-Anzeigeelementen.

Gegenstand der Erfindung sind ferner Dielektrika für Flüssigkristall-Anzeigeelemente mit mindestens zwei flüssigkristallinen Komponenten, von denen mindestens eine ein Fluorphenylcyclohexan der Formel (I) ist. Gegenstand der Erfindung sind schließlich elektrooptische Anzeigeelemente auf der Basis einer Flüssigkristallzelle, worin die Flüssigkristallzelle ein Dielektrikum mit einem Gehalt an einem Fluorphenylcyclohexan der Formel (I) enthält.

Die Fluorphenyl-trans-cyclohexane der Formel (I) sind entweder Ketone der Formel (Ia)

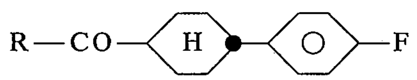

(Ia)

worin R die in Formel (I) angegebene Bedeutung besitzt, oder 4-(trans-4-Alkylcyclohexyl)-fluorbenzole der Formel (Ib)

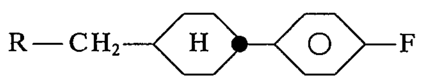

(Ib)

worin R die in Formel (I) angegebene Bedeutung besitzt. In allen erfindungsgemäßen Fluorphenylcyclohexanen der allgemeinen Formeln (I), (Ia) und (Ib) sind die jeweiligen Substituenten in den 1- und 4-Stellungen der Cyclohexanringe trans-ständig angeordnet; in den Formelbildern ist dies durch die schwarze Markierung auf der rechten Seite der Cyclohexanringe symbolisiert.

In der Regel besitzen die Ketone der Formel (Ia) eine niedrigere dielektrische Anisotropie als die 4-(4-Alkylcyclohexyl)-fluorbenzole der Formel (Ib). Die Ketone (Ia) finden daher bevorzugt Verwendung in Dielektrika für Flüssigkristall-Anzeigeelemente, die auf der Basis der Phänomene der dynamischen Streuung oder der Deformation aufgerichteter Phasen arbeiten. Die Verbindungen der Formel (Ib) werden entsprechend vorzugsweise in Dielektrika mit positiver dielektrischer Anisotropie verwendet, die in Anzeigeelementen auf der Basis der verdrillten nematischen Zelle eingesetzt werden. Soweit jedoch technische Kriterien wie die der dielektrischen Anisotropie oder auch beispielsweise der Löslichkeit bei der Auswahl der erfindungsgemäßen Verbindungen im Vergleich zu wirtschaftlichen Gesichtspunkten eine untergeordnete Rolle spielen, werden die Ketone (Ia) bevorzugt, da sie einfacher hergestellt werden können als die Verbindungen der Formel (Ib).

Der Substituent R in den Verbindungen der Formel (I) kann geradkettig oder verzweigt sein. Wenn R geradkettig ist, also Methyl, Äthyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl oder n-Heptadecyl bedeutet, besitzen die dadurch charakterisierten Verbindungen der Formel (I) in der Regel höhere Klärpunkte; ferner sind die Ausgangsmaterialien für ihre Synthese leichter zugänglich als die Ausgangsmaterialien für Verbindungen mit einem verzweigten Substituenten R. Diese Verbindungen, darunter die, in denen R geradkettiges Alkyl mit 1 bis 12 Kohlenstoffatomen, vorzugsweise 3 bis 10 Kohlenstoffatomen bedeutet, sind daher im Rahmen dieser Erfindung besonders bevorzugt. Gelegentlich sind jedoch auch Verbindungen der Formel (I) mit verzweigtem Substituenten R von Bedeutung, da diese manchmal bessere Löslichkeitseigenschaften in den üblichen flüssigkristallinen Grundmischungen aufweisen. Solche nicht geradkettigen Substituenten R enthalten vorzugsweise nicht mehr als eine Kettenverzweigung. Bevorzugte verzweigte Substituenten sind die, in denen die Kohlenstoffkette an dem der Gruppe Z benachbarten Kohlenstoffatom oder einem der beiden nächsten Kohlenstoffatome verzweigt ist. Von Bedeutung sind unter diesen solche verzweigten Gruppen, in denen sich an einer längeren Kohlenstoffkette in 1-, 2- oder 3-Stellung eine Methyl- oder Äthylgruppe befindet, beispielsweise Isopropyl, 1-Methylpropyl, 2-Methylpropyl, 1-Methylbutyl, 2-Methylbutyl, 3-Methylbutyl, 1-Methylpentyl, 1-Äthylpentyl, 2-Methylpentyl, 1-Methylhexyl, 2-Äthylhexyl oder 1-Methylheptyl.

Die Herstellung der erfindungsgemäßen 4-(4-Alkylcyclohexyl)-fluorbenzole der Formel (Ib) kann analog zu den in der DE-OS 2 636 684 beschriebenen 4-Alkyl-1-phenyl-cyclohexanen erfolgen.

Vorzugsweise werden die erfindungsgemäßen Fluorphenylcyclohexane jedoch hergestellt, indem man Cyclohexen und Fluorbenzol in Gegenwart einer Lewis-Säure mit einem Carbonsäurehalogenid der Formel (II)

$$R—CO—Hal \qquad (II)$$

worin R die vorstehend genannte Bedeutung besitzt und Hal Chlor oder Brom bedeutet, gegebenenfalls in Gegenwart eines inerten Lösungsmittels, bei einer Temperatur zwischen −50° und +100° umsetzt und gegebenenfalls in der zunächst erhaltenen Verbindung der Formel (Ia) die Carbonylgruppe in an sich bekannter Weise zu einer Methylengruppe reduziert.

Als Carbonsäurehalogenide der Formel (II) kommen vorzugsweise die Chloride in Frage, die aus den entsprechenden Fettsäuren in einfacher Weise, zum Beispiel durch Umsetzung mit Thionylchlorid hergestellt werden können.

Die im erfindungsgemäßen Verfahren bevorzugt verwendeten Lewis-Säuren sind Aluminiumchlorid, Bortrifluorid, Zinkchlorid, Eisentrichlorid oder Antimonpentachlorid. Aus wirtschaftlichen Gründen wird Aluminiumchlorid bevorzugt.

Als Lösungsmittel für das erfindungsgemäße Verfahren können prinzipiell die bei Friedel-Crafts-Reaktionen üblichen verwendet werden. Bevorzugt werden Tetrachlorkohlenstoff, Dichlormethan, 1,2-Dichloräthan, Schwefelkohlenstoff, Petroläther, n-Hexan oder Cyclohexan eingesetzt. Die Umsetzung kann jedoch auch in Abwesenheit eines zusätzlichen Lösungsmittels durchgeführt werden. In diesem Fall hat es sich als zweckmäßig erwiesen, einen Überschuß an Fluorbenzol zu verwenden.

Die Reaktion kann im Temperaturbereich von −50° bis +100° durchgeführt werden. Vorteilhaft wählt man zu Beginn der Umsetzung eine niedrigere Temperatur, vorzugsweise zwischen −30° und +10°, und läßt das Reaktionsgemisch sich gegen Ende auf Raumtemperatur erwärmen; gelegentlich ist es günstig, zur Vervollständigung der Umsetzung am Ende kurze Zeit bis zum Siedepunkt des verwendeten Lösungsmittels zu erwärmen. Die Aufarbeitung erfolgt in an sich üblicher Weise, bevorzugt durch Hydrolyse mit wäßriger Salzsäure bei Temperaturen zwischen −10° und +20°, Extraktion des gebildeten Ketons der Formel (Ia) mit einem geeigneten organischen Lösungsmittel, zum Beispiel Toluol, und fraktionierte Destillation des Extrakts unter vermindertem Druck. Die so hergestellten Ketone der Formel (Ia) können ohne weitere Reinigung als Komponenten flüssigkristalliner Dielektrika verwendet werden; falls eine Reinigung erwünscht ist, werden die entsprechenden Destillatfraktionen vorzugsweise aus Methanol oder Äthanol umkristallisiert.

Die Reduktion der Ketone (Ia) zu den entsprechenden 4-(4-Alkylcyclohexyl)-fluorbenzolen (Ib) erfolgt in an sich bekannter Weise, zum Beispiel mit Hydrazinhydrat und einem Alkalimetallhydroxid oder -alkoholat in einem hochsiedenden Lösungsmittel wie Diäthylenglykol oder Dimethylsulfoxid, mit Zink und Chlorwasserstoff in Diäthyläther oder mit amalgamiertem Zink in wäßriger Salzsäure. Nach an sich üblicher Aufarbeitung werden die so erhaltenen Verbindungen der Formel (Ib) durch fraktionierte Destillation unter vermindertem Druck oder durch Umkristallisieren aus einem geeigneten Lösungsmittel, zum Beispiel Äthanol oder Essigsäureäthylester, gereinigt.

Die erfindungsgemäßen Dielektrika bestehen aus zwei oder mehr Komponenten, darunter mindestens eine der Formel (I). Die weiteren Komponenten sind vorzugsweise nematische oder nematogene Substanzen aus den Klassen der Azobenzole, Azoxybenzole, Biphenyle, Schiffschen Basen, insbesondere Benzyliden-Derivate, Phenylbenzoate, Phenylcyclohexane, gegebenenfalls halogenierten Stilbene, Diphenylacetylen-Derivate, Diphenylnitrone und substituierten Zimtsäuren. Die wichtigsten als derartige weitere Komponenten in Frage kommenden Verbindungen lassen sich durch die Formel (V) charakterisieren:

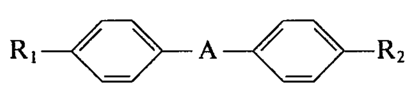 (V)

worin

A —CH=CH— 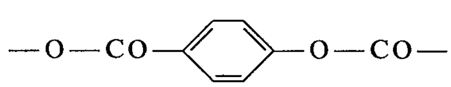

—CX'=CH— 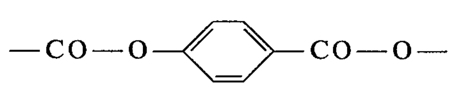

—CH=CX'— 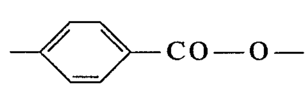

—C≡C— 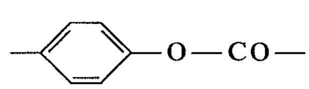

—N=N—     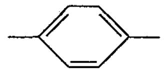—CO—S—

—N(O)=N—     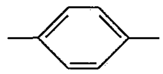—S—CO—

—N=N(O)—     —CH=N—

—O—CO—     —N=CH—

—CO—O—     —CH=N(O)—

—S—CO—     —N(O)=CH—

—CO—S—     oder eine C—C-Einfachbindung

bedeutet; wenn A —CO—O-, —O—CO- oder eine C—C-Einfachbindung bedeutet, kann einer der beiden Phenylringe auch durch einen trans-Cyclohexylring ersetzt sein; X′ bedeutet Halogen, vorzugsweise Cl. $R_1$ und $R_2$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten; weiterhin kann einer dieser Reste auch eine Cyano-, Nitro- oder Isonitrilgruppe bedeuten. Bei den meisten dieser Verbindungen sind $R_1$ und $R_2$ vorzugsweise verschieden, wobei einer der Reste meist eine Alkyl- oder Alkoxygruppe bedeutet. Aber auch eine große Zahl anderer Varianten der vorgesehenen Substituenten sind gebräuchlich. Viele solcher nematischen Substanzen sind im Handel erhältlich.

Die erfindungsgemäßen flüssigkristallinen Dielektrika enthalten in der Regel mindestens 1 und höchstens 35 Gewichtsteile eines oder mehrerer Fluorphenylcyclohexane der Formel (I). Bevorzugt werden solche Materialien, die 3 bis 30, vorzugsweise 5 bis 20 Gewichtsteile mindestens eines Fluorphenylcyclohexans der Formel (I) enthalten. Durch diese Zusätze wird die Viskosität der flüssigkristallinen Basismaterialien durchschnittlich um 10—50% gesenkt. Entsprechend werden die Schaltzeiten von mit den erfindungsgemäßen Dielektrika hergestellten Flüssigkristall-Anzeigeelementen verkürzt. Die Klärpunkte der erfindungsgemäßen Dielektrika sind zwar niedriger als die der flüssigkristallinen Basismaterialien. Bei geeigneter Wahl der Basismaterialien sowie der zugesetzten Menge des/der Fluorphenylcyclohexane der Formel (I) werden jedoch Gemische erhalten, deren Klärpunkte auch bei der im Dauerbetrieb der damit hergestellten Anzeigeelemente auftretenden Erwärmung nicht erreicht werden. Falls der Zusatz eines oder mehrerer Fluorphenylcyclohexane der Formel (I) zu einem flüssigkristallinen Basismaterial eine Mischung mit einem für die technische Anwendung zu tiefen Klärpunkt ergibt, kann dieser Effekt gegebenenfalls durch Zugabe von 1—30, vorzugsweise 2—20 Gewichtsteilen eines oder mehrerer Biphenylester der Formel (IV), eines oder mehrerer Hexahydroterphenyle nach der DE-OS 2 701 591 oder eines oder mehrerer der Cyclohexanderivate nach der DE-OS 2 800 553 kompensiert werden.

Durch geeignete Zusätze können die flüssigkristallinen Dielektrika nach der Erfindung so modifiziert werden, daß sie in allen bisher bekanntgewordenen Arten von Anzeigeelementen angewandt werden können. Derartige Zusätze sind dem Fachmann bekannt und sind in der einschlägigen Literatur ausführlich beschrieben. Beispielsweise können Substanzen zur Veränderung der dielektrischen Anisotropie und/oder der Orientierung der nematischen Phasen zugesetzt werden. Derartige Substanzen sind zum Beispiel in den DE-OS 2 209 127, 2 321 632 und 2 611 453 beschrieben.

Die folgenden Beispiele sollen die Erfindung erläutern, ohne sie zu begrenzen. In den Beispielen bedeutet F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

Zu einer Suspension von 160 g Aluminiumchlorid in 192 g Fluorbenzol wird unter Rühren und Kühlen auf −10° im Lauf von 2 Stunden ein Gemisch von 82 g Cyclohexen und 132 g n-Heptanoylchlorid getropft. Anschließend wird das Reaktionsgemisch bei +20° noch 16 Stunden gerührt und dann in ein Gemisch aus 300 g Eis und 300 ml rauchender Salzsäure gegossen. Die organische Phase wird abgetrennt, die wäßrige noch zweimal mit je 200 ml Toluol extrahiert, die vereinigten organischen Phasen über Calciumchlorid getrocknet und destilliert. Nach Abdestillation des überschüssigen Fluorbenzols und des Toluols wird der Rückstand unter vermindertem Druck (5—10 mm Hg) destilliert. Eine erste Fraktion von etwa 10% des Rückstandes wird als Vorlauf verworfen; das anschließende

abdestillierte 4-(trans-4-n-Heptanoylcyclohexyl)-fluorbenzol, das etwa 65% des Rückstandes ausmacht, wird mit der gleichen Volumenmenge Methanol aufgenommen und bei −20° kristallisiert; Ausbeute 73 g, F. 47°, K. −10°.

Analog werden erhalten:

4-(trans-4-Acetylcyclohexyl)-fluorbenzol,
4-(trans-4-Propionylcyclohexyl)-fluorbenzol, F. 53°, K. −20°,
4-(trans-4-n-Butyrylcyclohexyl)-fluorbenzol,
4-(trans-4-Isobutyrylcyclohexyl)-fluorbenzol,
4-(trans-4-n-Pentanoylcyclohexyl)-fluorbenzol, F. 35°, K. −30°,
4-(trans-4-(2-Methylbutyryl)-cyclohexyl)-fluorbenzol,
4-(trans-4-(3-Methylbutyryl)-cyclohexyl)-fluorbenzol,
4-(trans-4-n-Hexanoylcyclohexyl)-fluorbenzol,
4-(trans-4-(2-Methylpentanoyl)-cyclohexyl)-fluorbenzol,
4-(trans-4-(3-Methylpentanoyl)-cyclohexyl)-fluorbenzol,
4-(trans-4-(2-Methylhexanoyl)-cyclohexyl)-fluorbenzol,
4-(trans-4-(2-Äthylpentanoyl)-cyclohexyl)-fluorbenzol,
4-(trans-4-n-Octanoylcyclohexyl)-fluorbenzol,
4-(trans-4-n-Nonanoylcyclohexyl)-fluorbenzol, F. 54°, K. −10°,
4-(trans-4-n-(3-Äthylheptanoyl)-cyclohexyl)-fluorbenzol,
4-(trans-4-n-Decanoylcyclohexyl)-fluorbenzol, F. 58°, K. −20°,
4-(trans-4-n-Undecanoylcyclohexyl)-fluorbenzol,
4-(trans-4-n-Dodecanoylcyclohexyl)-fluorbenzol,
4-(trans-4-n-Tridecanoylcyclohexyl)-fluorbenzol,
4-(trans-4-n-Tetradecanoylcyclohexyl)-fluorbenzol, F. 60°, K. +5°,
4-(trans-4-n-Pentadecanoylcyclohexyl)-fluorbenzol,
4-(trans-4-n-Hexadecanoylcyclohexyl)-fluorbenzol,
4-(trans-4-n-Heptadecanoylcyclohexyl)-fluorbenzol,
4-(trans-4-n-Octadecanoylcyclohexyl)-fluorbenzol.

Beispiel 2

Eine Lösung von 58 g 4-(trans-4-n-Heptanoylcyclohexyl)-fluorbenzol, 45 g Kaliumhydroxid und 30 g Hydrazinhydrat in 200 ml Diäthylenglykol wird im Lauf von 3 Stunden von 100° auf 195° erwärmt. Nach dem Erkalten wird das Reaktionsgemisch mit 300 ml Wasser versetzt und mit 200 ml Dichlormethan extrahiert. Aus dem Extrakt werden das Lösungsmittel und mit diesem azeotrop die Restfeuchtigkeit abdestilliert und das zurückbleibende 4-(trans-4-n-Heptylcyclohexyl)-fluorbenzol aus Essigsäureäthylester umkristallisiert; Ausbeute 47 g, F. 35°, K. −15°.

Analog werden erhalten:

4-(trans-4-Äthylcyclohexyl)-fluorbenzol,
4-(trans-4-n-Propylcyclohexyl)-fluorbenzol, F. 32°, K. −40°,
4-(trans-4-n-Butylcyclohexyl)-fluorbenzol,
4-(trans-4-Isobutylcyclohexyl)-fluorbenzol,
4-(trans-4-n-Pentylcyclohexyl)-fluorbenzol, F. 36°, K. −25°,
4-(trans-4-(2-Methylbutyl)-cyclohexyl)-fluorbenzol,
4-(trans-4-(3-Methylbutyl)-cyclohexyl)-fluorbenzol,
4-(trans-4-n-Hexylcyclohexyl)-fluorbenzol,
4-(trans-4-(2-Methylpentyl)-cyclohexyl)-fluorbenzol,
4-(trans-4-(3-Methylpentyl)-cyclohexyl)-fluorbenzol,
4-(trans-4-(2-Methylhexyl)-cyclohexyl)-fluorbenzol,
4-(trans-4-(2-Äthylpentyl)-cyclohexyl)-fluorbenzol,
4-(trans-4-n-Octylcyclohexyl)-fluorbenzol,
4-(trans-4-n-Nonylcyclohexyl)-fluorbenzol, F. 39°, K. −5°,
4-(trans-4-(3-Äthylheptyl)-cyclohexyl)-fluorbenzol,
4-(trans-4-n-Decylcyclohexyl)-fluorbenzol, F. 46°, K. −5°,
4-(trans-4-n-Undecylcyclohexyl)-fluorbenzol,
4-(trans-4-n-Dodecylcyclohexyl)-fluorbenzol,
4-(trans-4-n-Tridecylcyclohexyl)-fluorbenzol,
4-(trans-4-n-Tetradecylcyclohexyl)-fluorbenzol, F. 57°, K. 15°,
4-(trans-4-n-Pentadecylcyclohexyl)-fluorbenzol,
4-(trans-4-n-Hexadecylcyclohexyl)-fluorbenzol,

4-(trans-4-n-Heptadecylcyclohexyl)-fluorbenzol,
4-(trans-4-n-Octadecylcyclohexyl)-fluorbenzol.

Die folgenden Beispiele betreffen flüssigkristalline Dielektrika nach der Erfindung:

### Beispiel 3

Eine Mischung aus

27% 4-(trans-4-n-Propylcyclohexyl)-benzonitril,
39% 4-(trans-4-n-Pentylcyclohexyl)-benzonitril,
13% 4-(trans-4-n-Pentylcyclohexyl)-4'-cyanobiphenyl und
21% 4-(trans-4-n-Nonylcyclohexyl)-fluorbenzol

hat eine nematische Phase im Temperaturbereich von −10° bis +55°. Die dielektrische Anisotropie ist +10,2, die Viskosität 20 cSt. Das entsprechende Dielektrikum sonst gleicher Zusammensetzung, aber ohne das erfindungsgemäße Fluorphenylcyclohexan hat eine Viskosität von 32 cSt.

Das erfindungsgemäße Dielektrikum ist hervorragend zur Verwendung in Anzeigeelementen auf der Basis der verdrillten nematischen Zelle geeignet.

### Beispiel 4

Eine Mischung aus

24% 4-(trans-4-n-Butylcyclohexyl)-benzonitril,
16,5% 4-Äthyl-4'-cyanobiphenyl,
12,0% 4-n-Propyloxy-4'-cyanobiphenyl,
17,5% 4-(trans-4-n-Pentylcyclohexyl)-4'-cyanobiphenyl,
7% 4-(trans-4-n-Pentylcyclohexyl)-benzoesäure-4'-cyanophenylester und
23% 4-(trans-4-n-Heptylcyclohexyl)-fluorbenzol

hat eine nematische Phase im Temperaturbereich von −6° bis +61°. Die dielektrische Anisotropie ist +12,5 und die Viskosität 32 cSt. Das entsprechende Dielektrikum sonst gleicher Zusammensetzung, aber ohne das erfindungsgemäße Fluorphenylcyclohexan hat eine Viskosität von 40 cSt.

Das erfindungsgemäße Dielektrikum ist gut geeignet für Anzeigeelemente auf der Basis der verdrillten nematischen Zelle, insbesondere auch in der Form von Matrix-Displays.

### Beispiel 5

Eine Mischung aus

47% 4-(trans-4-n-Propylcyclohexyl)-1-n-butyryloxybenzol,
19% 4-(trans-4-n-Butylcyclohexyl)-benzoesäure-(4-n-propylcyclohexyl)-ester,
14% 4-(trans-4-Äthylcyclohexyl)-benzoesäure-(4-n-propylcyclohexylester und
20% 4-(trans-4-Propionylcyclohexyl)-fluorbenzol

hat eine nematische Phase im Temperaturbereich von −5° bis +65°. Die dielektrische Anisotropie ist −0,2, die Viskosität 30 cSt. Das entsprechende Dielektrikum sonst gleicher Zusammensetzung, aber ohne das erfindungsgemäße Fluorphenylcyclohexan hat eine Viskosität von 36 cSt.

Dieses erfindungsgemäße Dielektrikum ist besonders gut geeignet für Anzeigeelemente, die auf der Basis der dynamischen Streuung arbeiten.

**Patentansprüche für die Vertragsstaaten CH, DE, FR, GB, IT, NL**

1. Fluorphenyl-trans-cyclohexane der Formel (I)

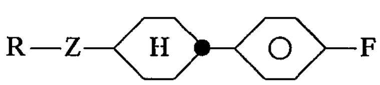

(I)

worin R eine Alkylgruppe mit 1 bis 17 Kohlenstoffatomen und Z entweder eine Carbonylgruppe oder eine Methylengruppe bedeutet.

2. Fluorphenylcyclohexane nach Anspruch 1, dadurch gekennzeichnet, daß R eine geradkettige Alkylgruppe ist.

3. Verfahren zur Herstellung von Fluorphenyl-trans-cyclohexanen der Formel (I)

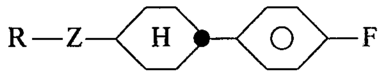 (I)

worin R eine Alkylgruppe mit 1 bis 17 Kohlenstoffatomen und Z entweder eine Carbonylgruppe oder eine Methylengruppe bedeutet, dadurch gekennzeichnet, daß Cyclohexen und Fluorbenzol in Gegenwart einer Lewis-Säure mit einem Carbonsäurehalogenid der Formel (II)

R—CO—Hal (II)

worin R die vorstehend genannte Bedeutung besitzt und Hal Chlor oder Brom bedeutet, gegebenenfalls in Gegenwart eines inerten Lösungsmittels, bei einer Temperatur zwischen −50° und +100° umsetzt und gegebenenfalls in der zunächst erhaltenen Verbindung der Formel (I), worin Z eine Carbonylgruppe bedeutet, diese in an sich bekannter Weise zu einer Methylengruppe reduziert.

4. Verwendung eines Fluorphenyl-trans-cyclohexans der Formel (I)

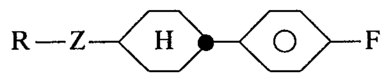 (I)

worin R eine Alkylgruppe mit 1 bis 17 Kohlenstoffatomen und Z entweder eine Carbonylgruppe oder eine Methylengruppe bedeutet, als Komponente eines flüssigkristallinen Dielektrikums für elektrooptische Anzeigeelemente.

5. Flüssigkristallines Dielektrikum mit mindestens 2 Komponenten, dadurch gekennzeichnet, daß es mindestens ein Fluorphenyl-trans-cyclohexan der Formel (I) enthält,

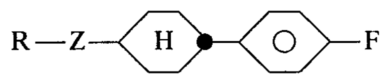

worin R eine Alkylgruppe mit 1 bis 17 Kohlenstoffatomen und Z entweder eine Carbonylgruppe oder eine Methylengruppe bedeutet.

6. Elektrooptisches Anzeigeelement auf der Basis einer Flüssigkristall-Zelle, dadurch gekennzeichnet, daß die Flüssigkristall-Zelle ein Dielektrikum nach Anspruch 5 enthält.

**Patentansprüche für den Vertragsstaat AT**

1. Verfahren zur Herstellung der Fluorphenyl-trans-cyclohexane der Formel (I)

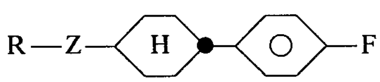 (I)

worin R eine Alkylgruppe mit 1 bis 17 Kohlenstoffatomen und Z entweder eine Carbonylgruppe oder eine Methylengruppe bedeutet, dadurch gekennzeichnet, daß Cyclohexen und Fluorbenzol in Gegenwart einer Lewis-Säure mit einem Carbonsäurehalogenid der Formel (II)

R—CO—Hal (II)

worin R die vorstehend genannte Bedeutung besitzt und Hal Chlor oder Brom bedeutet, gegebenenfalls in Gegenwart eines inerten Lösungsmittels, bei einer Temperatur zwischen −50° und +100° umsetzt und gegebenenfalls in der zunächst erhaltenen Verbindung der Formel (I), worin Z eine Carbonylgruppe bedeutet, diese in an sich bekannter Weise zu einer Methylengruppe reduziert.

2. Verwendung eines Fluorphenyl-trans-cyclohexans der Formel (I)

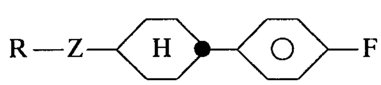 (I)

worin R eine Alkylgruppe mit 1 bis 17 Kohlenstoffatomen und Z entweder eine Carbonylgruppe oder eine Methylengruppe bedeutet, als Komponente eines flüssigkristallinen Dielektrikums für elektrooptische Anzeigeelemente.

3. Flüssigkristallines Dielektrikum mit mindestens 2 Komponenten, dadurch gekennzeichnet, daß es mindestens ein Fluorphenyl-trans-cyclohexan der Formel (I) enthält,

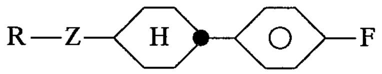

worin R eine Alkylgruppe mit 1 bis 17 Kohlenstoffatomen und Z entweder eine Carbonylgruppe oder eine Methylengruppe bedeutet.

4. Elektrooptisches Anzeigeelement auf der Basis einer Flüssigkristall-Zelle, dadurch gekennzeichnet, daß die Flüssigkristall-Zelle ein Dielektrikum nach Anspruch 3 enthält.

**Claims for the Contracting States CH, DE, FR, GB, IT, NL**

1. Fluorophenyl-trans-cyclohexanes of the formula (I)

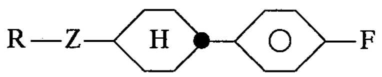

(I)

wherein R is an alkyl group with 1 to 17 carbon atoms and Z is either a carbonyl group or a methylene group.

2. Fluorophenylcyclohexanes according to Claim 1, characterised in that R is a straight-chain alkyl group.

3. Process for the preparation of fluorophenyl-trans-cyclohexanes of the formula (I)

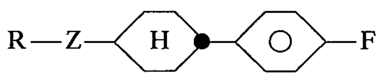

(I)

wherein R is an alkyl group with 1 to 17 carbon atoms and Z is either a carbonyl group or a methylene group, characterised in that cyclohexene and fluorobenzene are reacted with a carboxylic acid halide of the formula (II)

R—CO—Hal (II)

wherein R has the abovementioned meaning and Hal is chlorine or bromine, in the presence of a Lewis acid and if appropriate in the presence of an inert solvent, at a temperature between $-50°$ and $+100°$, and, in the initially obtained compound of the formula (I) wherein Z denotes a carbonyl group, this group is optionally reduced to a methylene group in a manner which is in itself known.

4. Use of a fluorophenyl-trans-cyclohexane of the formula (I)

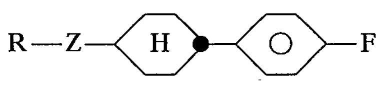

(I)

wherein R is an alkyl group with 1 to 17 carbon atoms and Z is either a carbonyl group or a methylene group, as a component of a liquid crystal dielectric for electrooptical display elements.

5. Liquid crystal dielectric with at least two components, characterised in that it contains at least one fluorophenyl-trans-cyclohexane of the formula (I)

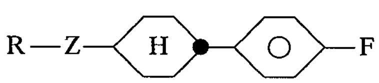

wherein R is an alkyl group with 1 to 17 carbon atoms and Z is either a carbonyl group or a methylene group.

6. Electrooptical display element based on a liquid crystal cell, characterised in that the liquid crystal cell contains a dielectric according to Claim 5.

**Claims for the Contracting State AT**

1. Process for the preparation of fluorophenyl-trans-cyclohexanes of the formula (I)

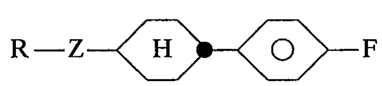

(I)

wherein R is an alkyl group with 1 to 17 carbon atoms and Z is either a carbonyl group or a methylene

group, characterised in that cyclohexene and fluorobenzene are reacted with a carboxylic acid halide of the formula (II)

$$R—CO—Hal \qquad (II)$$

wherein R has the abovementioned meaning and Hal is chlorine or bromine, in the presence of a Lewis acid and if appropriate in the presence of an inert solvent, at a temperature between $-50°$ and $+100°$, and, in the initially obtained compound of the formula (I) wherein Z denotes a carbonyl group, this group is optionally reduced to a methylene group in a manner which is in itself known.

2. Use of a fluorophenyl-trans-cyclohexane of the formula (I)

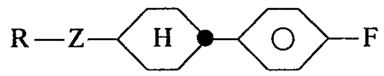

(I)

wherein R is an alkyl group with 1 to 17 carbon atoms and Z is either a carbonyl group or a methylene group, as a component of a liquid crystal dielectric for electrooptical display elements.

3. Liquid crystal dielectric with a least two components, characterised in that it contains at least one fluorophenyl-trans-cyclohexane of the formula (I)

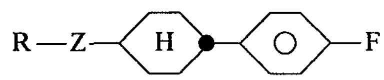

wherein R is an alkyl group with 1 to 17 carbon atoms and Z is either a carbonyl group or a methylene group.

4. Electrooptical display element based on a liquid crystal cell, characterised in that the liquid crystal cell contains a dielectric according to Claim 3.

**Revendications pour les Etats contractants CH, DE, FR, GB, IT, NL**

1. Fluorophényl-trans-cyclohexanes de formule I

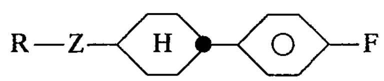

(I)

dans laquelle R représente un groupe alkyle en C1—C17 et Z représente un groupe carbonyle ou un groupe méthylène.

2. Fluorophénylcyclohexanes selon la revendication 1, caractérisés en ce que R représente un groupe alkyle à chaîne droite.

3. Procédé de préparation des fluorophényl-trans-cyclohexanes de formule I

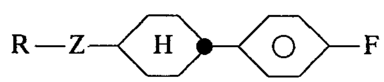

(I)

dans laquelle R représente un groupe alkyle en C1—C17 et Z représente un groupe carbonyle ou un groupe méthylène, caractérisé en ce que l'on fait réagir le cyclohexène et le fluorobenzène en présence d'un acide de Lewis avec un halogénure d'acide carboxylique de formule II

$$R—CO—Hal \qquad (II)$$

dans laquelle R a la signification indiquée ci-dessus et Hal représente le chlore ou le brome, éventuellement en présence d'un solvant inerte, à une température allant de $-50$ à $+100°C$ et le cas échéant, dans le composé de formule I obtenu en premier et dans lequel Z représente un groupe carbonyle, on réduit ce dernier, de manière connue en soi, en un groupe méthylène.

4. Utilisation d'un fluorophényl-trans-cyclohexane de formule I

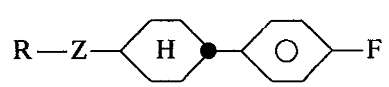

(I)

dans laquelle R représente un groupe alkyle en C1—C17 et Z représente un groupe carbonyle ou un groupe méthylène, en tant que composant d'un diélectrique à cristaux liquides pour éléments d'affichage électro-optiques.

5. Diélectrique à cristaux liquides à au moins deux composants, caractérisé en ce qu'il contient au moins un fluorophényl-trans-cyclohexane de formule

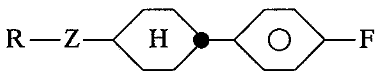

dans laquelle R représente un groupe alkyle en C1—C17 et Z représente un groupe carbonyle ou un groupe méthylène.

6. Elément d'affichage électro-optique à base d'une cellule à cristaux liquides, caractérisé en ce que la cellule à cristaux liquides contient un diélectrique selon la revendication 5.

**Revendications pour l'Etat contractant AT**

1. Procédé de préparation des fluorophényl-trans-cyclohexanes de formule I

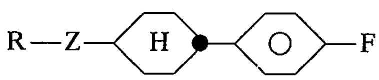

(I)

dans laquelle R représente un groupe alkyle en C1—C17 et Z représente un groupe carbonyle ou un groupe méthylène, caractérisé en ce que l'on fait réagir le cyclohexène et le fluorobenzène en présence d'un acide de Lewis avec un halogénure d'acide carboxylique de formule II

$$R-CO-Hal \qquad (II)$$

dans laquelle R a la signification indiquée ci-dessus et Hal représente le chlore ou le brome, éventuellement en présence d'un solvant inerte, à une température allant de −50 à +100°C et le cas échéant, dans le composé de formule I obtenu en premier et dans lequel Z représente un groupe carbonyle, on réduit ce dernier, de manière connue en soi, en un groupe méthylène.

2. Utilisation d'un fluorophényl-trans-cyclohexane de formule I

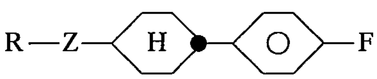

(I)

dans laquelle R représente un groupe alkyle en C1—C17 et Z représente un groupe carbonyle ou un groupe méthylène, en tant que composant d'un diélectrique à cristaux liquides pour éléments d'affichage électro-optiques.

3. Diélectrique à cristaux liquides à au moins deux composants, caractérisé en ce qu'il contient au moins un fluorophényl-trans-cyclohexane de formule

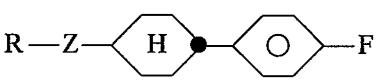

dans laquelle R repésente un groupe alkyle en C1—C17 et Z représente un groupe carbonyle ou un groupe méthylène.

4. Elément d'affichage électro-optique à base d'une cellule à cristaux liquides, caractérisé en ce que la cellule à cristaux liquides contient un diélectrique selon la revendication 3.